Europäisches Patentamt

European Patent Office

Office européen des brevets

(11) Veröffentlichungsnummer: **0 037 011**
**B1**

(12) **EUROPÄISCHE PATENTSCHRIFT**

(45) Veröffentlichungstag der Patentschrift: **07.08.85**

(21) Anmeldenummer: **81102009.8**

(22) Anmeldetag: **18.03.81**

(51) Int. Cl.⁴: **A 61 K 7/48, A 61 K 9/06**

(54) Salbengrundlagen für dermatologische und kosmetische Zwecke.

(30) Priorität: **19.03.80 DE 3010572**

(43) Veröffentlichungstag der Anmeldung:
**07.10.81 Patentblatt 81/40**

(45) Bekanntmachung des Hinweises auf die
Patenterteilung:
**07.08.85 Patentblatt 85/32**

(84) Benannte Vertragsstaaten:
**AT BE CH DE FR GB IT LI NL SE**

(56) Entgegenhaltungen:
**DE-B-1 134 478**
**GB-A- 714 795**
**US-A-4 151 272**

**Patents Abstracts of Japan, Band 3, Nr. 138**
**(C-64) 16. November 1979, Seite 161C64,**
**TOKYO (JP)**
**Remington's Pharmaceutical Sciences,15th Ed,**
**Mack Pub. (1975) S. 1247-48**
**Römpps Chemie-Lexikon, Neumüller,**
**Franckh'sche Verlag(1977),S.3780**

(73) Patentinhaber: **Süess, Hans R., Dr.**
**Choliweid**
**CH-4656 Starrkirch (CH)**

(72) Erfinder: **Süess, Hans R., Dr.**
**Choliweid**
**CH-4656 Starrkirch (CH)**

(74) Vertreter: **Kohler, Anton, Dr. et al**
**Dr.A.Kohler + M.Schroeder Patentanwälte**
**Franz-Joseph-Strasse 48**
**D-8000 München 40 (DE)**

**Beschreibung**

Die Erfindung betrifft Salbengrundlagen für dermatologische und kosmetische Zwecke auf der Basis von Vaseline® unter Zusatz von Siloxan. Die Salbengrundlagen sind zur kosmetischen oder medikamentösen Anwendung auf der gesunden, verletzten oder kranken Haut bestimmt.

Die Haut, das größte Organ des menschlichen Körpers, dient primär dessen Schutz gegen die Umgebung. Das Integument ist im wesentlichen drei schädigenden Einwirkungen ausgesetzt: UV-Strahlung, Chemikalien und zu trockene oder zu feuchte Umgebung. Dagegen ist die Haut zu schützen. Gegen UV-Strahlung läßt sich dies tun, indem man Sonnenexposition möglichst vermeidet und/oder Sonnenschutzpräparate anwendet, gegen zu trockene oder zu feuchte Umgebung und z.T. auch gegen Chemikalien schützt sie sich durch das von den Talgdrüsen gebildete Sebum und zu einem allerdings viel geringeren Teil durch das bei der Verhornung der Zellen gebildete Fett. Die Tätigkeit der Talgdrüsen ist hormonell gesteuert und nimmt mit dem Alter ab. Deshalb ist die Fettversorgung der Haut beim älteren Menschen ungenügend, woraus sich die Wichtigkeit einer vermehrten Fettzufuhr von außen ergibt. Das Hautfett wird nun durch hygienische Maßnahmen wie Waschen, Baden oder Duschen weitgehend entfernt und dies insbesondere bei den am meisten gewaschenen Hautpartien wie Hände und Gesicht. Bei geringer, z.B. durch Kälte bedingter, Luftfeuchtigkeit erleidet die ungeschützte Haut einen starken Feuchtigkeitsverlust—sie trocknet aus, und es können schmerzhafte Hautrisse entstehen. Andererseits entstehen bei langandauernder Einwirkung von Wasser, insbesondere wenn dessen Oberflächenspannung durch Seifen oder Syndets stark herabgesetzt ist, Hautschäden durch Mazeration (z.B. Waschfrauenhände).

Die kosmetische Industrie sucht Austrocknungszustände der Haut durch sogenannte Feuchtigkeitschremes (Moisturizer) zu beheben. Alle Cremes liegen als Öl-in-Wasser- oder als Wasser-in-Öl- bzw. Misch-Emulsionen vor. Sie enthalten Emulgatoren, welche das Durchdringen von Wasser und das Entfernen durch Waschen des aufgetrockneten Fettfilmes erleichtern. Diese Zubereitungen vermögen den transepidermalen Wasserverlust der Haut nur ungenügend zu unterbinden, und die erreichte Feuchthaltung der Haut ist unbefriedigend. Vaseline® hingegen vermag den Wasserverlust der Haut weitgehend zu unterbinden. Vaseline® ist ein eingetragenes Warenzeichen für aus Rohölen hergestelltes Petrolatum, das als Gelbe und Weiße Vaseline® im Handel ist (vergl. "Römpps Chemie-Lexikon", 1977, Seite 3780). Kligman (siehe z.B. "Cosmetics and Toiletries", *93* (4) 27 (1978) hat denn auch wiederholt darauf hingewiesen, daß dieses Kohlenwasserstoffgemisch der Vaseline® (ungeachtet, ob weiß, gelb oder rot) mit Abstand das beste Feuchthaltemittel (Moisturizer) sei; gleichzeitig ist er aber der Ansicht, daß diese Wirkung nicht auf einer Verminderung des transepidermalen Wasserverlustes durch Okklusion, sondern auf einer pharmakologischen Wirkung der Vaselin® auf die Haut beruhe. Vaselinfett ist jedoch kosmetisch höchst unbefriedigend, weil es zufolge seiner Zähigkeit schlecht in die Haut eindringt und ungenügend auf ihr haftet, so daß es mechanisch und durch Waschen leicht wieder entfernbar ist. Weiterhin läßt es sich wegen seiner Konsistenz nur schwierig dünn auftragen, so daß sich eine unangenehme fettige und klebrige Schicht bildet, die beim Arbeiten äußerst störend ist. Es ist bekannt, daß die Vaseline® neben einer gewissen hautreizenden Wirkung auch eine Epidermisverbreiterung (Akanthose) bewirken kann. Dies ist ein Hinweis auf die pharmakologische Wirkung der Vaseline®. Die erwähnten Eigenschaften kommen offenbar vorwiegend den unterhalb von 180°C bei 2 mbar siedenden Anteilen der Vaseline® zu (siehe z.B. Schaaf, "Problems der dermatologischen Grundlagenforschung" 1969, S. 105 ff.). Die reizarme Restfraktion läßt sich wegen ihrer Zähigkeit nur schlecht auf der Haut verteilen und ist somit kosmetisch unbefriedigend.

Aus "Lexikon der Hilfsstoffe für Pharmazie, Kosmetik und angrenzende Gebiete", (1971), Seite 615 bis 617, ist die Verwendung von Vaseline®, deren Hydrophobie nur von den Siliconen übertroffen wird, in Salbengrundlagen bekannt. Die Probleme der Hautreizung werden eingehend angesprochen. Obgleich angegeben wird, daß bestimmte, den Anforderungen der Arzneibücher entsprechende Vaselinen® keine toxischen, carcinogegen oder sonstwie schädigende Eigenschaften besitzen, ist bekannt, daß alle Vaselinen® des Handels, auch wenn sie Arzneibuchanforderungen entsprechen, zumindest latent hautreizend sein können, d.h. sie können unvermischt Hautverträglichkeit aufweisen, sobald aber das kolloidale System durch Zugabe flüssiger Stoffe, in denen Vaselinen® löslich sind, verändert wird, können niedrigmolekulare Anteile, z.B. $C_{10}$—$C_{18}$-Kohlenwasserstoffe, welche hautreizend sind, (vgl. "Cosmetics and Toiletries", *94*, (8) 41, (1979) sich im ausblutenden Zusatzstoff anreichern und zur bekannten stechenden Hautreizung Anlaß geben. Demgegenüber haben sich die erfindungsgemäß eingesetzten Vaselinfraktionen, aus denen die leicht flüchtigen Weißöle bis auf einen Gehalt von unter 20 Gew.% abgetrennt wurden, auch wenn sie in den speziellen Siloxanen gelöst sind und damit leichter in die Haut eindringen, als vollkommen reizfrei erwiesen. In "Römpps Chemie-Lexikon", 7. Auflage, (1976), S. 3223 ff. und "Kosmetik und Aerosole" in Seifen-Öle-Fette-Wachse, 100. Jg. Nr. 7/1974, S. 173 bis 177, werden ausschließlich lineare, praktisch nicht-flüchtige Dimethylsiloxane beschrieben, die bei Anwendung in Salbengrundlagen als wesentliche Bestandteile in dem auf der Haut verbleibenden Wirkstoffgemisch vorliegen, während die erfindungsgemäß eingesetzten speziellen Siloxane flüchtig sind und bei ihrer Anwendung nicht auf der Haut verbleiben, wobei nur der festhaftende Vaselinfilm in feiner Verteilung auf der Haut als Schutz vorliegt. Auch wird in der BE—B—1 134 478 eine Hautschutzalbe beschrieben, die aus 45 bis 60 Gew.% eines Dimethyl- und/oder Diphenylpolysiloxans mit einer Viskosität von 150 bis 350 Centistokes, 15 bis 30 Gew.% Zinkstearat und 25 Gew.% paraffinischer Salbengrundlage aufgebaut ist. Die dabei eingesetzten Siloxane sind nichtflüchtig und verbleiben auf der Haut, während die erfindungsgemäß eingesetzten Siloxane flüchtig sind, d.h. ein geschmeidiger Auftrag des Mittels auf die Haut wird begünstigt, und nach Abdampfen des flüchtigen Siloxan-Lösungsmittels verbleibt auf der Haut ein festhaftender Vaselinfilm in feiner Verteilung. In Remington's Pharmaceutical Science 15. Ausgabe, Mack Publishing Co., Eaton, Pa. USA, (1975), S. 1247—48, sind als Salbengrundlagen übliches Petrolatum NF als Petrolatum USP wiedergegeben. Gemäß der Erfindung werden jedoch nur spezielle Vaselinfraktionen, deren Gehalt an Weißölen unter 20 Gew.% liegt, eingesetzt.

Nach der US—A—4 151 272 ist ferner ein Antiperspirantstift bekannt, der aus wachsartigen Fettalkohol, Adstringens, flüchtigem Silicon und bestimmten polyäthoxylierten Fattalkoholen aufgebaut ist. Einerseits muß ein Antiperspirantstift andere Anforderungen erfüllen als eine Salbengrundlage, in der keinerlei Adstringens vorgesehen ist, und darüber hinaus wird in einem derartigen Stift weder Vaseline® nach die erfindungsgemäß eingesetzte spezielle Vaselinfraktion mit einem Gehalt an Weißölen unter 20 Gew.% angewendet.

Die Aufgabe der Erfindung besteht darin, eine auf der Haut gut verteilbare und fest haftende physiologisch unbedenkliche Salbengrundlage zu schaffen, die reizfrei ist und auch bei häufigem Waschen einen lang anhaltenden Schutz bietet.

Es wurde nun gefunden, daß man die bisherigen Nachteile vermeiden kann, wann man bestimmte Vaselinfraktionen mit bestimmten Mengen spezieller, geeigneter, physiologisch unbedenklicher Lösungsmittel vermischt.

Gegenstand der Erfindung sind Salbengrundlagen für dermatologische und kosmetische Zwecke auf der Basis von Vaseline® unter Zusatz von Siloxan, gekennzeichnet durch einen Gehalt an

a) 10 bis 90 Gew.% Vaselinfraktionen, deren Gehalt an Weißölen unter 20 Gew.% liegt, und

b) 90 bis 10 Gew.% Octamethylcyclotetrasiloxan, Decamethylcyclopentasiloxan und/oder Hexamethyldisiloxan als Lösungsmittel.

Das Vermischen der angegebenen Mengen Lösungsmittel mit der Vaselinfraktion erfolgt zweckmäßig bei leicht erhöhter Temperatur. Beim Abkühlen entsteht ein leicht verstreichbares Präparat.

Bevorzugt liegt das Verhältnis von Vaseline® zu Lösungsmittel bei 30 bis 70 Gew.% zu 70 bis 30 Gew.%. Vorzugsweise wird eine Vaselinfraktion verwendet, deren Gewichtsverhältnis von festen zu bei 20 bis 25°C flüssigen Anteilen bei über 3, insbesondere 5 bis 7 liegt. Nach einer besonders bevorzugten Ausführungsform sind in der erfindungsgemäßen Salbengrundlage hochschmelzende Mikrokristallinwachse enthalten.

Zweckmäßig weist die vorliegende Salbengrundlage als zusätzliche Komponente physiologisch verträgliche niedere Alkohols auf.

Die Vaselinkomponente der erfindungsgemäßen Salbengrundlagen besteht aus physiologisch unbedenklichen Vaselinfraktionen, deren Gehalt an Weißölen unter 20 Gew.% liegt. Die Weißöle umfassen die niedrigsiedenden, niedrigviskosen, hautreizende Wirkung ausübenden Bestandteile der Vaseline®. Nach Abtrennung der Weißöle, beispielsweise durch Destillation, erhält man somit reizfreie Vaselinfraktionen. Vorzugsweise enthalten die Vaselinfraktionen keine Anteile mit weniger als 20 Kohlenstoffatomen im Molekül. Zur Herstellung der erfindungsgemäß eingesetzten Vaselinfraktionen können beliebige Vaselinen® verwendet werden. Der Einsatz der vorstehend beschriebenen Vaselinfraktionen, die nur einen geringen Gehalt an Weißölen aufweisen oder aus denen durch Destillation die Weißöle auf den angegebenen Gehalt abgetrennt wurden und somit nur eine geringe Wahrscheinlichkeit einer möglichen Hautreizung besteht, führt in Verbindung mit den gemäß der Erfindung verwendbaren physiologisch unbedenklichen Lösungsmitteln zu stabilen, opaken, dermatologisch günstigen und kosmetisch ansprechenden Zubereitungen.

Es besonders zweckmäßig, solche Vaselinfraktionen zu verwenden, die ein Gewichtsverhältnis von festen zu bei 20 bis 25°C flüssigen Anteilen von über 3 aufweisen. Insbesonere soll das Verhältnis von festen zu bei 20 bis 25°C flüssigen Anteilen 10:1 nicht übersteigen. Vorzugsweise erhält man derartige Vaselinfraktionen durch Extraktion des Rohdestillats mit halogernierten, insbesondere chlorierten Kohlenwasserstoffen.

Die erfindungsgemäßen Salbengrundlagen enthalten als Lösungsmittel für die angegebenen Vaselinfraktionen Hexamethyldisiloxan, Octamethylcyclotetrasiloxan und/oder Decamethylcyclopentasiloxan. Diese Lösungsmittel sind physiologisch verträglich und bilden mit der Vaselinfraktion in der Wärme, d.h. bei oder über dem Schmelzbereich der Vaselinfraktion, klare Lösungen. Vorzugsweise sind die Lösungsmittel beim Abkühlen, z.B. unterhalb von 35°C nur noch beschränkt in den Vaselinfraktionen löslich, so daß selbenartige Gemische entstehen. Zudem sind die genannten Lösungsmittel flüchtig und verbleiben nicht auf der Haut. Unter Flüchtigkeit ist zu verstehen, daß die Lösungsmittel aufgrund ihrer geringen Verdampfungswärme innerhalb kurzer Zeit von der Haut abdunsten. Die Lösungsmittel können sowohl einzeln als auch im Gemisch verwendet werden. Die verwendeten Siloxane dienen ausschließlich als Lösungsmittel, um eine gute Verteilung der Vaseline® auf der Haut zu erreichen, verbleiben aufgrund der Flüchtigkeit jedoch nicht auf der Haut und hinterlassen nach ihrer Verdampfung einen schützenden Vaselinfilm auf der Haut. Die Flüchtigkeit der angewendeten Lösungsmittel ist neben ihrer Inertheit für die Wirksamkeit der Präparate und den damit erzielbaren Hautschutz von größter Bedutung.

Nach einer bevorzugten Ausführungsform verwendet man als Vaselinkomponente eine Vaseline®, deren flüssige Anteile gegenüber den festen stark vermindert sind. Dadurch wird ein noch besserer Hautschutz erhalten, da diese Präparate auch unter der Einwirkung von Detergentien einen dauerhaften Hautschutz bilden.

Handelsübliche, den Anforderungen der Arzneibücher entsprechende Vaselinen® bestehen aus ungefähr gleichen Teilen von bei 20 bis 25°C flüssigen und von über 60°C schmelzenden festen Anteilen. Wählt man nun ein Gewichtsverhältnis von festen zu flüssigen Anteilen von über 3, vorzugsweise jedoch 5 bis 7, so erhält man zähe auf der Haut kaum verstreichbare Massen, welche keine den Arzneibüchern entsprechenden Vaselinen® mehr sind. Durch Zusatz von Octamethylcyclotetrasiloxan, Decamethylcyclopentasiloxan und/oder Hexamethyldisiloxan erhält man jedoch gut verstreichbare, festhaftende Massen. Besonders günstig ist die Kombination von 20 bis 40 Gew.% der angegebenen Siloxane mit 80 bis 60 Gew.% Vaselinfraktion mit einem Gewichtsverhältnis von festen zu flüssigen Anteilen von 5 bis 7.

Ein bei Anwendung dieser Zubereitungen auf der Haut verbleibender Fettglanz kann durch Erhöhung des Anteils der hochschmelzenden Bestandteile der Vaseline® durch Zufügung von hochschmelzenden Mikrokristallinwachsen praktisch beseitigt werden, ohne die gute Verstreichbarkeit des Präparates zu beeinträchtigen. Hochschmelzende Mikrokristallinwachse mit einem Schmelzbereich von 85 bis 95°C liefern hervorragende Ergebnisse. Durch Zusatz von hochschmelzenden Mikrokristallinwachen (Fp ca. 90°C) im Ausmasse von 5 bis 15 Gew.%, bezogen auf die Summe von Vaselinfraktion und Lösungsmittel, erhält man weiße, opake Massen, die auf der Haut verstrichen gut haften und kaum noch Glanz hinterlassen. Dies ist überraschend, werden doch Mikrokristallinwachse sonst zur Glanzerhöhung, insbesondere bei Parafinwachsen, eingesetzt.

Gegebenenfalls können die erfindungsgemäßen Zubereitungen noch weitere physiologisch verträgliche Zusätze enthalten. Dabei sind Zusätze in geringen Mengen, z.B. bis zu 5%, geeignet. Bevorzugt wird die Zugabe von physiologisch unbedenklichen niedrigen Alkoholen, wie z.B. Äthyl- und Isopropylalkohol. Werden bei der Herstellung geringe Mengen derartiger Zusätze zugegeben, so erhält man besonders leicht verstreichbare, kosmetisch ansprechende Zubereitungen, die matt und nicht glänzend auftrocknen und zudem einen oftmals erwünschten Abkühlungseffekt aufweisen. Ferner können weitere übliche Zusätze in den erfindungsgemäßen Zubereitungen enthalten sein, soweit sie physiologisch unbedecklich sind und mit den verwendeten Lösungsmitteln verträglich sind. Beispiele derartiger Zusätze sind UV-Adsorber, Geruchstoffe und Verdickungsmittel wie z.B. Ceresin, Ozokerit, Aluminiumstearat, Polyvinlylstearat und Polyvinylpyrrolidonderivate, sodann, zur Modifikation des Hautgefühls, Isopropylester von Fettsäuren wie Isopropylmyristat und Lanolinderivate.

Die Menge der inagesamt in der erfindungsgemäßen Salbengrundlage vorliegenden Zusätze soll vorzugsweise 20 Gew.%, bezogen auf die Summe von Vaselinfraktion und Siloxanlösungsmittel, nicht überschreiten.

Die aus den Vaselinfraktionen und den angegebenen Siloxanlösungsmitteln in den aufgeführten Mengenverhältnissen sowie gegegenenfalls weiteren Zusätzen aufgebauten Zubereitungen sind hervorragende Salbengrundlagen und ausgezeichnete Hautpflege- und Hautschutzpräparate, die sich auf der Haut leicht verstreichen lassen und auch in den tieferen Teil der Hornhaut, welcher bei den weißrassigen Menschen aus etwa 16 Zellschichten besteht, eindringen. Dies ist begreiflich, weil die Abmessungen der gelösten Vaselinmoleküle einige Zehnerpotenzen unter der Größe der dispersen Phase von Emulsionen liegen. Nach Abdunsten des Lösungsmittels bleibt ein Kohlenwasserstoffgemisch zurück, das sich auch durch mehrmaliges Waschen der Haut mit Seife oder Syndets nicht mehr vollständig entfernen läßt. Solchermaßen geschützte Haut setzt dem Durchgang von Wasser in beiden Richtungen und somit auch dem Eindrigen von wasserlöslichen Noxen großen Widerstand entgegen. Da das Vaselinfett unverseifbar ist und chemische Noxen meist in wäßriger Lösung, oft bei höheren pH-Werten und/oder in Gegenwart von Netzmitteln vorliegen (z.B. Hauschaltswaschmittel oder thioglykolsäurehaltige Kaltwellpräparate), entfalten die erfindungsgemäßen Zubereitungen eine ausgezeichnete Schutzwirkung für die Haut.

Da die erfindungsgemäßen wasserfreien Präparate keinerlei Konservierungsmittel benötigen, lassen sich auch durch solche Wirkstoffe bedingte Allergien vermeiden. Durch den Einsatz von Hexamethyldisiloxan, Octamethylcyclotetrasiloxan und/oder Decamethylcyclopentasiloxan als Lösungsmittel, die olfaktorisch kaum wahrnehmbar sind, lassen sich bei entsprechender Wahl der Vaselinfraktion praktisch geruchlose Präparate erhalten, die keinerlei Geruchskorrigentien bedürfen. Dadurch lassen sich allergische und phototoxische Erscheinungen wie sie bei der Anwendung von Riechstoffen auftreten können, vermeiden. Dies ist besonders wichtig, wenn erfindungsgemäß hergestellte Zubereitungen unter Zufügung von geeigneten UV-Absorbern als Sonnenschutzmittel dienen sollen. Dazu eignen sie sich hervorragend, weil die Vaseline® an sich schon Sonnenschutzeignachaften hat und well das emulgatorfreie Präparat beim Baden nicht ausgewaschen und vom Sand mechanisch kaum abgetragen wird. Man erhält damit lange wirksame Sonnenschutzpräparate, was vom Standpunkt des Lichtschutzes der Haut von großer Wichtigkeit ist.

Sodann ist die Geruchsfreiheit von Handschutzpräparaten ein Erfordernis, wenn die Hände danach mit Lebensmitteln in Berührung kommen, weil die Möglichkeit einer unerwünschten Geruchsübertragung bei parfümierten Produkten besteht. Die erfindungsgemäßen Präparate eignen sich auch in besonderer Weise als Gewerbeschutzcreme.

Beispiel 1

| | |
|---|---:|
| Vaseline® (oberhalb von 180°C bei 2 mbar siedende Fraktion) | 60 g |
| Octamethylcyclotetrasiloxan | 40 g |
| | 100 g |

Die beiden Komponenten werden erwämt bis eine klare Lösung entstanden ist, hernach wird kaltgerührt.

Beispiel 2

| | |
|---|---:|
| Vaseline® (Fp 58°C) | 65 g |
| Decamethylcyclopentasiloxan | 35 g |
| | 100 g |

Die Komponenten werden erwärmt bis eine klare Lösung entstanden ist, anschließend wird kaltgerührt.

Beispiel 3

| | |
|---|---:|
| Vaseline® (oberhalb von 180°C bei 2 mbar siedende Fraktion) | 60 g |
| Octamethylcyclotetrasiloxan | 35 g |
| Isopropanol | 5 g |
| | 100 g |

Die Komponenten werden erwärmt bis eine klare Lösung entstanden ist, anschließend wird kaltgerührt.

Sämtliche in den vorstehenden Beispielen hergestellte Präparate ergeben hervorragenden Hautschutz.

Beispiel 4

| | |
|---|---:|
| Vaselin®festanteil (Fp über 60°C) | 600 g |
| Vaselin®flüssiganteil (bei 20 bis 25°C flüssig) | 100 g |
| Mikrokristallinwachs, Fp 88 bis 91°C | 80 g |
| Octamethylcyclotetrasiloxan | 320 g |
| | 1100 g |

Die ersten 3 Anteile werden in einem mit einem Rückflußkühler versehenen, heizbaren Gefäß bei ca. 95°C geschmolzen, und nachdem eine homogene Lösung entstanden ist, wird das Octamethylcyclotetrasiloxan beigefügt und unter schwachem Rühren abgekühlt. Nach Stehenlassen während 24 Std. bildet sich ein reinweißes Lipogel, das sich auf der Haut leicht verstreichen läßt und die damit behandelten Hände sehr stark hydrophobisiert. Die anzuwendende Menge ist gering: 0,3 bis 0,5 g sind für einen Hautschutz während eines ganzes Arbeitstages im allgemeinen ausreichend.

Beispiel 5

| | |
|---|---:|
| Vaselin®festanteil (Fp über 60°C) | 500 g |
| Vaselin®flüssiganteil (bei 20 bis 25°C flüssig) | 100 g |
| Mikrokristallinwachs, Fp 88 bis 91°C | 90 g |
| Octamethylcyclotetrasiloxan | 310 g |
| | 1000 g |

Die Harstellung erfolgt in gleicher Weise wie bei Beispiel 4. Die entstandene Zubereitung ergibt auf der Hand verstrichen ein besondere angenehmes Hautgefühl.

Beispiel 6

| | |
|---|---|
| Vaselin®festanteil (Fp über 60°C) | 500 g |
| Vaselin®flüssiganteil (bei 20 bis 25°C flüssig) | 100 g |
| Mikrokristallinwachs, Fp 88 bis 91°C | 110 g |
| Decamethylcyclopentasiloxan | 390 g |
| | 1100 g |

Die Herstellung erfolgt in gleicher Weise wie bei Beispiel 4. Die erhaltene Zubereitung läßt sich auf der Haut leicht zu einem dünnen, matt auftrocknenden Film verstreichen, der auch bei häufigem Waschen auf der Haut als Schutz verbleibt.

Beispiel 7

| | |
|---|---|
| Vaseline® (oberhalb von 180°C bei 2 mbar siedende Fraktion) | 60 g |
| Hexamethyldisiloxan | 40 g |
| | 100 g |

Die Komponenten werden erwärmt, bis eine klare Lösung entstanden ist, anschließend wird kaltgerührt.

Das erhaltene Präparat ist auf der Haut gut verstreichbar und liefert einen lang anhaltenden Hautschutz.

**Patentansprüche**

1. Salbengrundlagen für dermatologische und kosmetische Zwecke auf der Basis von Vaseline® unter Zusatz von Siloxan, gekennzeichnet durch einen Gehalt an
a) 10 bis 90 Gew.% Vaselin®fraktionen, deren Gehalt an Weißölen unter 20 Gew.% liegt, und
b) 90 bis 10 Gew.% Octamethylcyclotetrasiloxan, Decamethylcyclopentasiloxan und/oder Hexamethyldisiloxan als Lösungsmittel.

2. Salbengrundlagen nach Anspruch 1, dadurch gekennzeichnet, daß das Verhältnis von Vaseline® zu Lösungsmittel 30 bis 70 Gew.% zu 70 bis 30 Gew.% beträgt.

3. Salbengrundlagen nach Anspruch 1 oder 2, dadurch gekennzeichnet, daß die Vaselin®fraktion keine Anteile mit weniger als 20 Kohlenstoffatomen im Molekül aufweist.

4. Salbengrundlagen nach Anspruch 1 bis 3, dadurch gekennzeichnet, daß als zusätzliche Komponente hochschmelzende Mikrokristallinwachse enthalten sind.

5. Salbengrundlagen nach Anspruch 1 bis 4, dadurch gekennzeichnet, daß als zusätzliche Komponente physiologisch verträgliche niedere Alkohole enthalten sind.

**Revendications**

1. Bases de onguent à usage dermatologique et cosmétique à base de Vaseline® additionnée de Siloxan, caractérisées en ce qu'elles contiennent
a) de 10 a 90% en poids de fractions de Vaseline® dont la teneur en huiles blanches est inférieure à 20% en poids, et
b) de 90 a 10% en poids d'octaméthylcyclotétrasiloxan, décaméthylcyclopentasiloxan et/ou héxaméthyldisiloxan comme solvant.

2. Bases de onguent selon la revendication 1, caractérisées en ce que le rapport de la Vaseline® et le solvant est entre 30 à 70% en poids et 70 à 30% en poids.

3. Bases de onguent selon la revendication 1 ou 2, caractérisées en ce que la fraction de Vaseline® ne contient aucun ingrédient comprenant moins de 20 atomes de carbone dans la molécule.

4. Bases de onguent selon les revendications 1 à 3, caractérisées en ce qu'elles contiennent comme composants additifs des cires microcristallines à point de fusion haute.

5. Bases de onguent selon les revendications 1 à 4, caractérisées en ce qu'elles contiennent comme composants additifs des alcools inférieurs et compatibles physiologiquement.

**Claims**

1. Ointment base for dermatologic and cosmetic uses on the basis of vaseline® with the addition of siloxane, characterized by containing

a) 10 to 90 percent by weight of vaseline® fractions containing white oils in an amount below 20 percent by weight and

b) 90 to 10 percent by weight of octamethylcyclotetrasiloxane, decamethylcyclopentasiloxane and/or hexamethyldisiloxane as solvent.

2. Ointment base according to claim 1, characterized in that the ratio of vaseline® to solvent is 30:70 percent by weight to 70:30 percent by weight.

3. Ointment base according to claim 1 or 2, characterized in that the vaseline® fraction contains no constituents having less than 20 carbon atoms per molecule.

4. Ointment base according to claims 1 to 3, characterized in that high melting microcrystalline waxes are contained as additional component.

5. Ointment base according to claims 1 to 4, characterized in that physiologically tolerable lower alcohols are contained as additional component.